# DEMANDE DE BREVET EUROPEEN

(11) **EP 0 736 298 A1**
(43) Date de publication de la demande: **09.10.1996**
(21) Numéro de dépôt: 96400757.9
(22) Date de dépôt: 05.04.1996
(51) Int. Cl.: A61K 7/40

(54) **Shampoing antiparasitaire**

(30) Priorité: 07.04.1995 FR 9504198
(71) Demandeur: Benwaiche, Joseph, 64200 Arcangues (FR)
(72) Inventeur: Benwaiche, Joseph, 64200 Arcangues (FR)
(74) Mandataire: Sabatier, Marc

(57) **Abrégé**

Shampooing antiparasitaire caractérisé en ce que pour un litre de la préparation de base suivante :
- Lauryethersulfate de sodium 2,47 à 4,12 Kg;
- Stabilisant de mousse désépaississant 0,33 à 0,55 Kg;
- Agent tensio actif 0,24 à 0,41 Kg;
- Chlorure de sodium 0,12 à 0,20 Kg;
- Eau quantité suffisante pour 1 litre

on ajoute les composants suivants :
- Acide acétique 18,75 à 31,25 g;
- Formol 2,25 à 3,75 g;
- Chlorure de sodium 45 à 75 g;
- Eau 1 litre.

## Description

La présente invention concerne un shampooing antiparasitaire plus particulièrement destiné au traitement des insectes et surtout des acariens tels que poux ou puces, dans les cheveux ou poils, mais également aoûtats ou tiques en ce qui concerne les animaux. Dans ce domaine, à l'heure actuelle, il existe sur le marché un produit préférentiellement employé et qui est la pyrithione.

La pyrithione est en fait une désignation abrégée pour le -pyridinethiol -1- oxyde. C'est en fait un fongicide et un bactéricide contre les pellicules et les insectes.

Un tel composant entre dans la composition d'un shampooing antiparasitaire plus connu sous l'appellation commerciale MARIE-ROSE qui est citée exclusivement à titre d'exemple illustratif de l'art antérieur.

Ce produit donne des résultats généralement satisfaisants, mais il a pu être constaté que lors d'applications répétées pour un traitement intensif de longue durée, dans des cas difficiles, les insectes ou acariens parasites présentent un phénomène d'accoutumance à ce produit et deviennent plus résistants, d'où une plus grande difficulté à les exterminer.

La composition du shampooing connu précité est la suivante :
- Pyrethrines naturelles 0,30 g;
- Butoxyde de piperonyle 1,00 g;
- Excipient QSP 100 g;
- Colorant E124

C'est donc de façon à éviter les phénomènes d'accoutumance précités que l'invention propose un shampooing antiparasitaire, au moins aussi efficace que ceux de l'art antérieur, mais mettant en oeuvre des composants nouveaux au sens qu'ils n'avaient à ce jour encore jamais été introduits dans ce type de shampooing.

A cet effet l'invention concerne un shampooing antiparasitaire caractérisé en ce que pour un litre de la préparation de base suivante :
- Lauryethersulfate de sodium 2,47 à 4,12 Kg
- Stabilisant de mousse désépaississant 0,33 à 0,55 Kg
- Agent tensio actif 0,24 à 0,41 Kg
- Chlorure de sodium 0,12 à 0,20 Kg
- Eau quantité suffisante pour 1 litre
on ajoute les composants suivants :
- Acide acétique 18,75 à 31,25 g
- Formol 2,25 à 3,75 g
- Chlorure de sodium 45 à 75 g
- Eau 1 litre.

Selon cette formulation, s'agissant du stabilisant de mousse désépaississant, il sera employé un produit connu sous la marque "Comperlan KD" qui est en fait un mono, di ou polyalkylol amides d'acide gras, avant tout utilisé comme facteur donnant de la consistance aux préparations cosmétiques ou pharmaceutiques.

En ce qui concerne le laurylethersulfate de sodium, il sera employé un produit connu sous la marque "Tescapon N40" ayant de bonnes propriétés lavantes et émulsionnantes.

En ce qui concerne l'agent tensio actif il sera employé un produit connu sous la marque "RICINION" constituant un agent surgraissant obtenu par condensation d'oxyde d'éthylène sur chaîne ricinoleique pour former un liquide huileux soluble dans l'eau et l'acool éthylique.

Afin de rendre agréable l'utilisation du shampooing selon l'invention, celui-ci renferme 37,5 à 62,5 g d'un alcoolat de lavande, pour la formulation précitée.

Selon des proportions préférentielles des composants, pour un litre de la préparation de base suivante :
- Lauryethersulfate de sodium 3,3 Kg
- Stabilisant de mousse désépaississant 0,440 Kg
- Agent tensio actif 0,330 Kg
- Chlorure de sodium 0,165 Kg
- Eau quantité suffisante pour 1 litre
on ajoute les composants suivants :
- Acide acétique 25 g
- Formol 3 g
- Chlorure de sodium 60 g
- Eau 1 litre.

Egalement selon une proportion préférentielle, il sera ajouté à cette composition 50 ml d'un alcoolat de lavande.

## Revendications

1. Shampooing antiparasitaire caractérisé en ce que pour un litre de la préparation de base suivante :
- Lauryethersulfate de sodium 2,47 à 4,12 Kg;
- Stabilisant de mousse désépaississant 0,33 à 0,55 Kg;
- Agent tensio actif 0,24 à 0,41 Kg;
- Chlorure de sodium 0,12 à 0,20 Kg;
- Eau quantité suffisante pour un litre
on ajoute les composants suivants :
- Acide acétique 18,75 à 31,25 g;
- Formol 2,25 à 3,75 g;
- Chlorure de sodium 45 à 75 g;
- Eau 1 litre.

2. Shampooing selon la revendication 1, caractérisé en ce qu'il contient en outre 37,5 à 62,5 g d'un alcoolat de lavande.
